# DEMANDE DE BREVET EUROPEEN

(11) **EP 3 456 228 A1**
(43) Date de publication de la demande: **20.03.2019**
(21) Numéro de dépôt: 18194266.5
(22) Date de dépôt: 13.09.2018
(51) Int. Cl.: A47K 10/42, B65D 83/04, B65D 85/36, A61F 15/00

(54) **ADAPTATEUR POUR RECIPIENT TUBULAIRE**

(30) Priorité: 14.09.2017 BE 201705653
(71) Demandeur: Smidt, Sephane, 2970 's Gravenwezel (BE)
(72) Inventeur: Smidt, Sephane, 2970 's Gravenwezel (BE)
(74) Mandataire: Office Kirkpatrick

(57) **Abrégé**

L'invention concerne un accessoire facilitant la saisie par l'homme de produits contenus dans un récipient tubulaire, en particulier, un adaptateur destiné à être monté sur un récipient tubulaire comportant un fond et une ouverture de passage de produits contenus dans le récipient. L'adaptateur est agencé pour, après renversement du récipient, devenir le cuilleron d'une cuiller dont le récipient est le manche

## Description

L'invention concerne un accessoire facilitant la saisie par l'homme de produits contenus dans un récipient tubulaire.
Certains produits sont conditionnés dans des emballages tubulaires, de section carrée, rectangulaire ou cylindrique, relativement rigides. C'est notamment le cas de certains produits en vrac ou ayant plus ou moins une forme de disques empilés. Ces produits peuvent être alimentaires, comme des chips apéritives, desbiscuits ou des bonbons,ou non-alimentaires comme des disques de cotons à démaquiller ou des comprimés de médicament.

En général, l'ouverture de tels emballages ou récipients tubulaires se situe à l'une des extrémités du tube, qui possède une hauteur supérieure à sa section. Si les premiers produits sont relativement faciles à extraire du tube, l'inaccessibilité de la main de l'homme à la portion inférieure du récipient rendent les produits suivants difficile à attraper. La main doit en outre glisser le long de l'intérieur du tube, qui, pour certains produits, peut être gras, ce qui n'est pas hygiénique.

Confronté à ce problème, l'utilisateur incline habituellement le récipient tubulaire de façon à permettre aux produits de glisser par gravité vers l'ouverture, afin de pouvoir en saisir, un ou plusieurs, avec sa main. Toutefois, cette solution n'est pas toujours adéquate, car le risque de sortir une quantité trop importante de produits du tube est grand.

On peut également utiliser un instrument tel qu'une pince ou une cuiller pour attraper les produits dans la portion inférieure du récipient, inaccessible à la main de l'homme. Toutefois, cela nécessite d'avoir à sa disposition l'instrument adéquat. En outre, le maniement d'un tel instrument, inséré en profondeur dans un récipient tubulaire, n'est pas toujours aisé, et peut abimer les produits si ceux-ci sont fragiles.

C'est pourquoi, le demandeur a cherché un moyen d'accéder aux produits contenus dans le fond d'un récipient tubulaire, et par conséquent difficilement accessible par la main de l'homme, qui résoudrait les problèmes mentionnés ci-dessus.

A cet effet, la présente invention concerne un adaptateur agencé pour être monté sur un récipient tubulaire comportant un fond et une ouverture de passage de produits contenus dans le récipient, l'adaptateur étant agencé pour, après renversement du récipient, devenir le cuilleron d'une cuiller dont le récipient est le manche.

Un récipient tubulaire doit être considéré ici comme un récipient ayant une section et une hauteur telles qu'une portion de son espace intérieur adjacente à son fond est inaccessible à la main de l'homme. La section peut avoir n'importe quelle forme. Elle peut par exemple être circulaire, carrée ou rectangulaire.

L'invention ne s'applique qu'à des récipients tubulaires destinés à contenir des produits solides et non pulvérulents et pas de pâte, c'est-à-dire de produit pâteux non emballé. Le récipient tubulaire est en outre ici rigide, c'est-à-dire que sa forme tubulaire se conserve, que le récipient soit vide ou plein, et qu'une légère pression sur le tube n'en affecte pas la forme.

Il faut également ici admettre qu'un cuilleron d'une cuiller est agencé pour recevoir des produits que la main de l'homme peut saisir. Le cuilleron peut être lui-même envisagé comme un récipient, une coupelle de réception de produits.

On comprendra aisément que l'invention permet de transformer un récipient tubulaire avec l'adaptateur de l'invention en une véritable cuiller de saisie de produits, bien que les produits soient au départ à l'intérieur du manche (le récipient) de la cuiller dont l'adaptateur est devenu le cuilleron. La cuiller de saisie de produit peut également être comprise comme une coupelle de présentation de produits.

En d'autres termes, l'adaptateur de l'invention est agencé pour coopérer avec le récipient, de façon à former une cuiller dont le manche est constitué du récipient tubulaire et le cuilleron est constitué de l'adaptateur.

Grâce à l'adaptateur de l'invention, la saisie des produits contenus dans le récipient se fait de façon hygiénique, la main ne devant plus être introduite dans le récipient.

L'adaptateur comporte une ouverture d'introduction des produits à recueillir dans l'adaptateur. Cette ouverture d'introduction des produits est agencée pour correspondre, au moins en partie, à l'ouverture de passage de produits du récipient tubulaire sur lequel l'adaptateur est voué à être monté.

Avantageusement, l'adaptateur comprend des moyens pour enserrer le récipient tubulaire. Ces moyens peuvent par exemple être une bague ayant un diamètre à peine supérieur au tour du récipient tubulaire et qui peut être glissé autour du récipient tubulaire, de préférence proche de l'ouverture du récipient. Une telle bague peut être un anneau fermé ou partiellement ouvert.

Avantageusement encore, l'adaptateur comprend des moyens de blocage des produits à une distance déterminée au-delà de l'ouverture de passage de produits, afin d'éviter la chute des produits en dehors du cuilleron.

L'invention sera mieux comprise à la lecture de la description suivante d'une forme de réalisation préférée de l'invention, en référence au dessin en annexe sur lequel
- la figure 1 est une vue en perspective d'un adaptateur selon l'invention ;
- la figure 2 est une vue sous un autre angle de l'adaptateur de la figure 1 ;
- la figure 3 est une vue en perspective de l'adaptateur de la figure 1 installé sur un récipient tubulaire ;
- la figure 4 est une vue en perspective d'une autre forme de réalisation de l'adaptateur de l'invention, et
- la figure 5 est une vue en persepective d'une autre forme de réalisation de l'adapteteur de l'invention.

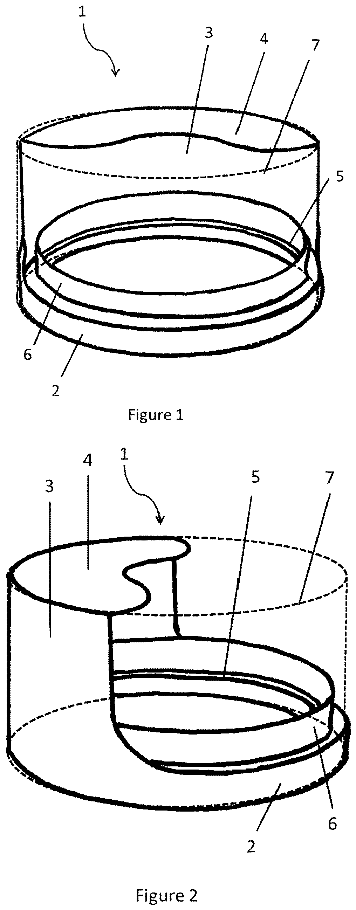

En référence aux figures 1 et 2, un adaptateur 1, dont la forme s'inscrit dans un cylindre 7, comprend, à une extrémité du cylindre, une bague 2 d'enserrage, à l'autre extrémité, une portion de disque 4, et, reliant les deux extrémités, une paroi 3 couvrant une partie de la surface du cylindre, ici la longueur de cylindre sur un peu moins de la moitié de son pourtour. La partie du cylindre non couverte par la paroi 3 définit une ouverture de saisie des produits contenus dans le récipient. Sur la paroi intérieure de la bague 2 est ici disposé un épaulement de butée 5. Un anneau 6 de fermeture est ici également accolé à la bague 2 d'enserrage, du côté intérieur au cylindre.

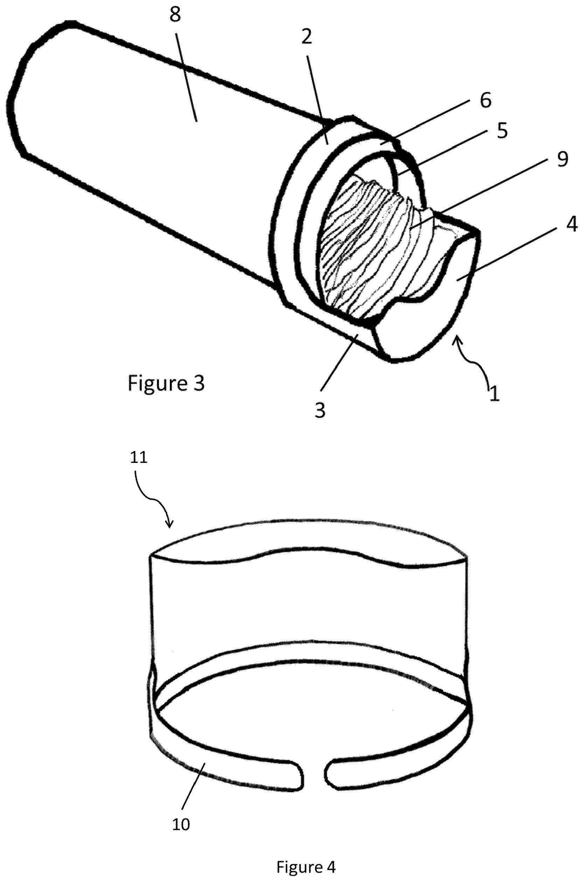

En référence à la figure 3, l'adaptateur 1 est emboité sur un récipient tubulaire 8 ouvert à une extrémité et contenant ici des chips 9. La bague 2 d'enserrage est placée autour du récipient 8, au niveau de son ouverture et définit une ouverture d'introduction des produits à recueillir dans l'adaptateur qui correspond à l'ouverture de passage des produits du récipient tubulaire 8.

En pratique, un utilisateur, après l'avoir ouvert, insère l'extrémité du récipient tubulaire 8 dans la bague 2 d'enserrage de l'adaptateur 1, jusqu'à ce que l'extrémité du récipient tubulaire soit en contact avec l'épaulement de butée 5. Le diamètre intérieur de la bague d'enserrage 2 est identique au diamètre extérieur du récipient tubulaire 8, ce qui permet à l'adaptateur 1 d'exercer une légère pression sur le pourtour du récipient 8 afin d'y être solidairement attaché.

Ainsi installé, l'adaptateur 1 et le récipient 8 forment une sorte de cuiller dont la paroi 3 et la portion de disque 4 forment le cuilleron de réception de chips 9. La partie du cylindre 7 non couverte par la paroi 3 est l'ouverture de saisie de produits contenus dans le récipient. Lors de l'inclinaison du récipient 8, des chips 9 glissent dans le cuilleron en traversant l'ouverture de passage des produits du récipient 8 et l'ouverture d'introduction des produits de l'adaptateur. L'utilisateur peut alors facilement en saisir le nombre qu'il souhaite, sans se salir les mains, ni renverser de produits.

L'épaulement de butée 5 n'est pas un élément essentiel de l'adaptateur 1. Il est néanmoins un moyen efficace et simple de blocage du coulissement de l'adaptateur 1 le long du récipient tubulaire 8, permettant de maintenir constante la taille du cuilleron.

L'adaptateur 1 comprend ici également un anneau 6 de fermeture. Cet anneau 6, accolé à la bague 2 d'enserrage, a le même diamètre extérieur que le récipient 8. Bien que cet anneau de fermeture ne soit pas non plus un élément essentiel de l'adaptateur, il permet, dans le cas où le récipient 8 serait fourni avec un couvercle, d'y replacer ce couvercle, sans avoir à retirer l'adaptateur. L'adaptateur 1 comporte ainsi des moyens agencés pour recevoir un capot d'obturation du récipient tubulaire 8, afin d'en assurer l'étanchéité si besoin.

La forme de l'adaptateur 1 et en particulier de la bague 2 peuvent également être agencées de façon à ce que l'adaptateur puisse être placé sur le récipient tubulaire 8 non ouvert et ayant déjà un couvercle. Par exemple, la base de la bague 2 peut comprendre un élargissement lui permettant d'être emboité sur un récipient tubulaire muni d'un couvercle. La bague 2 pourrait ainsi présenter trois sections consécutives de diamètres différents, une première section, à son extrémité, ayant le diamètre du couvercle du récipient tubulaire, une deuxième section intermédiaire ayant le diamètre du récipient tubulaire et une troisième section étant l'épaulement de butée 5 ayant un diamètre inférieur à celui du récipient tubulaire 8.

La paroi 3 a ici été décrite comme étant une partie de cylindre, qui s'inscrit dans la continuité du récipient tubulaire sur lequel l'adaptateur est placé. On peut toutefois envisager que cette paroi ait une autre forme, s'approchant plus par exemple de la forme hémisphérique d'une cuiller.

Il serait également envisageable, d'utiliser l'adaptateur 1 lui-même pour refermer le récipient tubulaire 8. Par exemple, l'extrémité du cylindre 7 opposée à la bague 2 d'enserrage pourrait être un disque complet, et, en l'absence de l'épaulement de butée 5, l'adaptateur 1 pourrait être coulissé le long du récipient 8 jusqu'à ce que le disque vienne obstruer l'ouverture du récipient tubulaire 8, au contact de ce dernier.

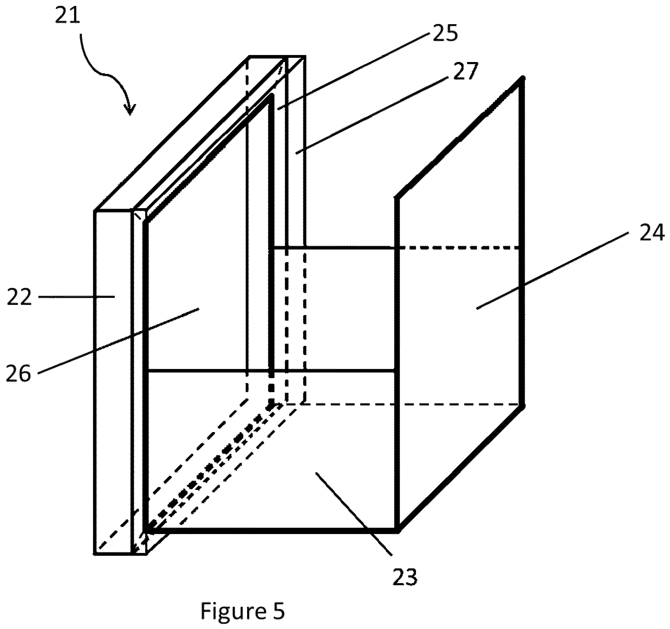

Alternativement, en référence à la figure 5, un adaptateur 21, de section carrée comporte une bague 22 d'enserrage, un réceptacle 23 et une paroi 24 de blocage ayant ici la même surface que l'ouverture 26 de passage des produits définie par la bague d'enserrage 22. La bague d'enserrage 22 comprend un épaulement 25 et un rebord de retour 27. L'adaptateur 21 est agencé pour être emboité sur un récipient tubulaire de section carrée au niveau de la bague 22, jusqu'à venir en butée contre l'épaulement 25. Lors de l'inclinaison du récipient tubulaire, les produits qui y sont contenus glissent à travers l'ouverture 26 jusque dans le réceptacle 23. Leur progression est arrêtée par la paroi 24. Les dimensions de la paroi 24 de blocage peuvent être définies pour correspondre exactement à la section intérieure du récipient tubulaire sur lequel l'adaptateur 21 est destiné à être emboité. Ainsi, après utilisation, l'adaptateur peut être déboité du récipient tubulaire, retourné et inséré à l'intérieur du récipient tubulaire, de façon à ce que la paroi 24 en obstrue complètement l'ouverture, la bague 2 servant alors d'épaulement de butée et le rebord de retour 27 servant à enserrer le récipient tubulaire dans cette configuration « fermée ». Cette utilisation implique évidemment que le récipient tubulaire ne soit pas rempli à ras bord, mais présente l'avantage de pouvoir combiner la fonction de cuilleron dans une première configuration et la fonction de bouchon ou couvercle dans une seconde configuration. L'adaptateur 21 est ainsi agencé pour être réversiblement un moyen d'obturation d'un récipient tubulaire.

La bague 2 d'enserrage ne se limite pas aux formes décrite plus haut et pourrait être conçue différemment. Par exemple, comme représenté sur la figure 4, la bague 10 d'enserrage de l'adaptateur 11 peut être un anneau discontinu, dont le diamètre est légèrement inférieur au diamètre du récipient tubulaire sur lequel l'adaptateur 11 est destiné à être installé. La bague 10 doit par conséquent être étirée pour épouser le pourtour du récipient, ce qui a pour effet d'augmenter la force de serrage de la bague 10 autour du récipient. Quand il n'est pas utilisé, l'adaptateur pourrait ainsi être glissé à l'intérieur du récipient sur lequel il est destiné à être emboité.

On peut également envisager d'autres systèmes pour fixer l'adaptateur sur un récipient tubulaire, comme des pièces faisant intervenir un adhésif repositionnable, ou une bague souple serrable grâce à des bandes Velcro®. Les moyens d'enserrage peuvent être fabriqués avec un diamètre fixe, adaptés à certains récipients tubulaires précis, ou éventuellement avec une certaine flexibilité de diamètre, permettant ainsi une utilisation plus universelle.

L'adaptateur peut être fabriqué dans n'importe quel matériau lui assurant une rigidité suffisante pour son utilisation. En particulier, l'adaptateur peut être fabriqué en plastique, en silicone voire en carton. Pour un usage alimentaire, l'adaptateur est fabriqué dans un matériau homologué pour le contact alimentaire, par exemple du polypropylène. L'utilisation du silicone peut permettre de fabriquer un adaptateur rétractable, c'est-à-dire compactable, ce qui permettrait un gain de place lors du stockage. L'adaptateur de l'invention n'est cependant pas limité à un usage alimentaire.

De même, les produits contenus dans le récipient sur lequel l'adaptateur est destiné à être emboité n'ont pas nécessairement une forme de disque, mais peuvent être des petits éléments en vrac, comme des bonbons ou des trombones.

## Revendications

1. Adaptateur (1; 11 ; 21) pour récipient tubulaire (8) comportant un fond et une ouverture d'introduction de produits (9) et une ouverture de saisie de produits (9), l'adaptateur (1 ; 11 ; 21) étant agencé pour, après montage sur un récipient tubulaire et renversement dudit récipient, devenir le cuilleron d'une cuiller dont le récipient (8) est le manche, **caractérisé par le fait que** l'adaptateur comprend une bague (2) d'enserrage présentant un épaulement de butée (5; 25) pour, après montage, bloquer le coulissement de l'adaptateur (1; 11; 21) sur le récipient tubulaire (8) qu'il enserre.

2. Adaptateur selon la revendication 1, dans lequel l'ouverture d'introduction des produits à recueillir dans l'adaptateur est définie par la bague (2) d'enserrage.

3. Adaptateur selon l'une des revendications 1 et 2, comprenant des moyens (4 ; 24) de blocage des produits à une distance déterminée au-delà de l'ouverture de passage de produits.

4. Adaptateur selon la revendication 3, dans lequel les moyens (4 ; 24) de blocage des produits à une distance déterminée au-delà de l'ouverture de passage de produits est une portion de disque.

5. Adaptateur selon l'une des revendications 1 et 4, comprenant des moyens (6) agencés pour recevoir un capot d'obturation du récipient tubulaire (8).

6. Adaptateur selon la revendication 5, dans lequel les moyens (6) agencés pour recevoir un capot d'obturation du récipient tubulaire (8) est un anneau (6) de fermeture accolé à la bague (2) d'enserrage.

7. Adaptateur selon la revendication 6, dans lequel l'anneau (6) de fermeture a le même diamètre que le récipient tubulaire (8) pour pouvoir y placer le couvercle du récipient (8).
